# EUROPEAN PATENT SPECIFICATION

(11) **EP 0 405 344 B1**
(45) Date of publication and mention of the grant of the patent: **01.09.1993**
(21) Application number: 90111831.5
(22) Date of filing: 22.06.1990
(51) Int. Cl.: C07C 217/74, A61K 31/135

(54) **2-Amino-1,2,3,4,-tetrahydronaphthalene derivatives with cardiovascular activity, process for their preparation and pharmaceutical compositions containing them**
2-Amino-1,2,3,4-Tetrahydronaphthalin-Derivate mit kardiovaskularer Wirkung, Verfahren zu ihrer Herstellung und pharmazeutische Zusammensetzungen, die sie enthalten
Dérivés de 1-Amino-1,2,3,4-tetrahydronaphthalène avec activité cardiovasculaire, procédé pour leur préparation et compositions pharmaceutiques les contenent

(30) Priority: 27.06.1989 IT 2099689
(43) Date of publication of application: 02.01.1991
(73) Proprietor: CHIESI FARMACEUTICI S.p.A., I-43100 Parma (IT)
(72) Inventor: Chiesi, Paolo, I-43100 Parma (IT); Bongrani, Stefano, I-43100 Parma (IT); Delcanale, Maurizio, I-43100 Parma (IT); Servadio, Vittorino, I-43100 Parma (IT)
(74) Representative: Bianchetti, Giuseppe

(56) References cited:
- EP-A- 0 209 275
- EP-A- 0 211 721
- EP-A- 0 273 017
- FR-A- 2 528 422
- US-A- 2 776 993
- JOURNAL OF MEDICINAL CHEMISTRY, vol. 24, no. 7, 1981, Washington, US, pages 835 - 839; R. J.GORCZYNSKI et al, "N-Aralkyl substitution of 2-amino-5,6- and -6,7-dihydroxy-1,2,3,4-tetrahydronaphthalenes. 1. Cardiac and pressor/depressor activities."
- JOURNAL OF MEDICINAL CHEMISTRY, vol. 20, no. 7, 1977, Washington, US, pages 978 - 981; H. E. SMITH et al, "AGONIST EFFECTS OF BETA-PHENETHYLAMINES ON THE NORADRENERGIC CYCLIC ADENOSINE 3',5'-MONOPHOSPHATE GENERATING SYSTEM IN RAT LIMBIC FOREBRAIN. STEREOISOMERS OF P-HYDROXYNORE PHEDRINE"

## Description

The present invention refers to compounds having cardiovascular activity belonging to the 2-amino-1,2,3,4-tetrahydronaphthalene type, to a process for their preparation and to pharmaceutical compositions containing them.

Cardiac insufficiency or cardio-circulatory failure is a major sanitary problem all over the world.

The classical therapy of this disease has since many years relied upon the use of cardiac glycosides and diuretics.

More recently also peripheral vasodilators have been used.

Many patients, however are not sensitive or are poorly sensitive to said therapies and, as a consequence, the pharmacological treatment of cardiac failure is still considered unsatisfactory, asking therefore for the search of new cardioactive drugs able to act on the main pathogenetic factors of the cardiocirculatory failure, namely the decrease of myocardial contractility and the impairments in peripheral blood flow.

It has now been found that the compound of formula (I) 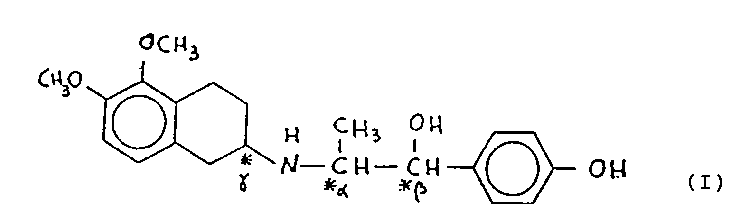
is endowed with inotropic and vasodilating activities so as to be advantageously used in the therapy of cardiocirculatory failure.

The above compound of formula (I) has three asymmetric carbon atoms in the positions marked by an asterisk and it should be understood that the present invention includes both the single enantiomers or diastereoisomers and the mixtures thereof, as well as the pharmaceutically acceptable salts of all these compounds.

The derivatives of 2-aminotetrahydronaphthalene (or 2-aminotetraline) are a class of compounds exhibiting interesting pharmacological properties because of their capacity of binding to the different receptors of the orthosympathetic system.

The 5,6 and 6,7 - dihydroxy derivatives of 2-aminotetraline contain in particular, fixed in a semi-rigid structure, the dopamine moiety which has in recent years given rise to a remarkable amount of research efforts.

Dopaminergic drugs may have in fact different therapeutic uses: drugs stimulating the post-synaptic dopamine receptors in the Central Nervous System (CNS) may be effective against Parkinson's disease; drugs acting as agonists of dopamine autoreceptors, always in the CNS, may be used as antipsychotics.

Dopaminergic receptors are however present also on the peripheral sympathetic terminations so that, particularly in the last years, many research efforts have been paid to dopaminergic structure having peripheral cardiovascular activity.

There are many structure-activity studies of 2-aminotetraline derivatives, whose results gave rise to different hypothesis on the importance and on the effects of the configuration of the C₂ carbon, of the optimal substitution on the amine, of the kind and number of the substituents on the aromatic ring and of changes of other parameters.

From these studies it is evident that 2-aminotetralines are rather heterogeneous as far as their pharmacological effects and their mechanism of action are concerned, the latter involving, besides both central and peripheral dopaminergic receptors, also adrenergic receptors both alpha and beta.

Also in this class, such as in other classes of sympathicomimetics, it is possible to notice how even very small structural differences may result in remarkable effects on the degree or on the kind of activity, depending on various interdependent factors such as the kind and position of substituents, conformation, interactions with the receptor site, metabolic pathway.

Many 2-aminotetraline derivatives are known and disclosed in patents and scientific papers.

The closest structural analogues of the compound (I) of the invention were first disclosed by Gorczynski R. J. et al in J.Med.Chem. 1981,24,835-839. The compound n° 14 of said paper is the structural closest analogue of the compound (I) and it has been therefore used as reference compound.

The 2-aminotetraline derivatives disclosed in EP-A-209275, EP-A-273017 and EP-A-211721 have structural differences in comparison with the compound I, which are significant under the pharmacological point of view.

In fact, both EP-A-209275 and EP-A-273017 disclose 6,7-dihydroxyderivatives; in the case of the latter application, said derivatives are moreover trisubstituted on the aromatic ring of the tetraline moiety.

The compounds of EP-A-211721, besides having structural differences, exhibit a completely different pharmacological action, namely lipolytic activity.

Finally, 5,6-dihydroxy derivatives of 2-aminotetraline having a potent and selective beta-2 stimulating adrenergic activity and which may be used as bronchodilators are known from GB-A-2123410 in the applicant's name.

The compounds of the present invention can be prepared by reductive amination of 5,6-dimethoxy-2-tetralone (II) with the appropriate primary amine (III) (or the salts thereof), according to the following reaction scheme :

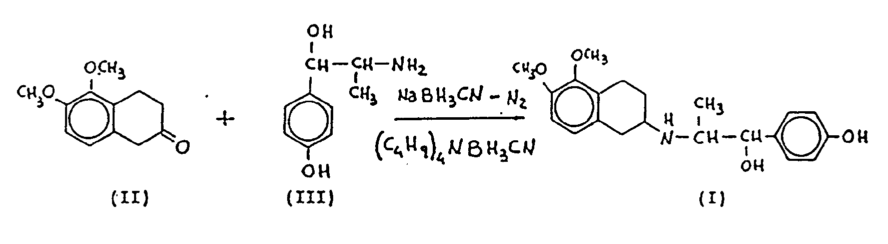

The condensation reaction is generally carried out in polar solvents such as dioxane or lower alcohols, preferably methanol or ethanol, at temperatures ranging from 0°C to 40°C, preferably under nitrogen atmosphere, at a pH adjusted around to neutral by addition of an acid, such as HCl or CH₃COOH, or of a base, such as triethylamine or pyridine.

The simultaneous reduction is effected by means of alkali hydrides, preferably sodium or lithium cyanoborohydrides or a mixture of sodium cyanoborohydride and tetrabutylammonium cyanoborohydride in a 4:1 to 1:1 by weight ratio.

Alternatively, reduction can be carried out with hydrogen in the presence of suited catalysts, such as Pt or Pd/C, under reduced pressure or at room pressure.

The compounds are recovered from the reaction mixture by per se known methods and are generally transformed into a mineral acid addition salt, preferably with hydrochloric acid, and crystallized as such.

Compound (I) can be prepared in form of a single stereoisomer or as couple of epimers, by condensation of 5,6-dimethoxy-2-tetralone (II) with a p-OH-norephedrine stereoisomer of known configuration and optionally by subsequent separation of the epimers obtained by crystallization or by chromatography.

The intermediates used in the preparation of the compounds are known and, if not commercially available, are prepared according to methods known in literature. Particularly, the stereoisomers of p-hydroxynorephedrine have been prepared by the processes described by Smith H.E. et al in J.Med. Chem. 20(7), 978(1977).

Compounds (I) can also be prepared according to a different process, as shown in the following reaction scheme : 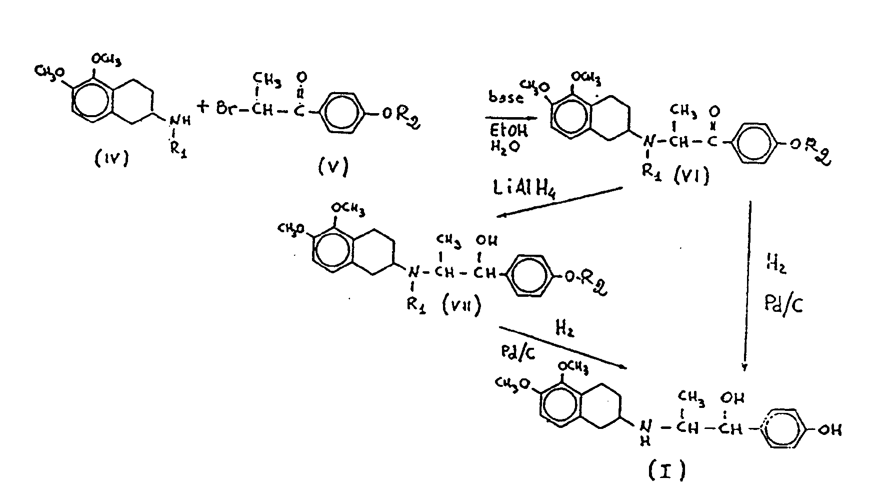
wherein R₁ and R₂ are H, CH₂-C₆H₅ or other easily cleavable protective group.

The condensation reaction of aminotetralin with the bromoketone is generally carried out in polar solvents, such as water, dioxane or lower alcohols, and preferably in ethanol or in an ethanol-water mixture, in the presence of an acid binding agent such as triethylamine or pyridine. The reaction may be carried out also in the absence of solvents at such a temperature to allow the partial or complete melting of the reaction mixture. The subsequent reduction can be effected by hydrogenation in the presence of suited catalysts, such as Pt or Pd/C or with alkali or alkali-earth metal hydrides and subsequent hydrogenolytic debenzylation.

This process proved to be particularly advantageous to obtain the compounds of the invention in a given configuration. The threo-isomers, with respect to the α and β carbon atoms may be obtained, for instance, by condensation of an aminotetralin (IV), in which R₁ is benzyl or a group having a similar steric hindrance, easly cleavable, with bromoketone (V) and subsequent reduction. The presence of the benzyl group or of a group of similar steric hindrance, allows to obtain a stereospecific reduction of aminoketones (VI) so as to attain the threo form of aminoalcohols (VII), according to a modification of the process described by Van Dijk J. e Moed H.D. in Recueil 78,22,1959. The erythro isomers, always with respect to the α and β carbon atoms, are obtained by chemical or catalytic reduction of aminoketones (VI) wherein R₁=H. Compounds having a given configuration with respect to carbon γ can be obtained starting from the suitable aminotetraline enantiomer (IV), obtained either by enantioselective synthesis or by known separation methods. Compounds (VI) are novel and are a further object of the invention, as useful intermediates for the preparation of compounds (I).

### EXAMPLE 1

### Erythro-5,6-dimethoxy-2[[2-(4-hydroxyphenyl)-2-hydroxy-1-methylethyl]amino]1,2,3,4-tetrahydronaphthalene hydrochloride (Ia).

A solution of 25 g (0.122 mole) of (±)-4-hydroxynorephedrine hydrochloride in 326 ml of methanol, adjusted to pH -7 with 5% KOH is slowly added to a solution containing 35.41 g (0.171 mol.) of 5,6-dimethoxy-2-tetralone in 346 ml of methanol, cooled to about 12°C and under nitrogen atmosphere. The reaction mixture is stirred under nitrogen for about 20 hours at room temperature; thereafter a mixture of 11.84 g of NaBH₃CN and 5.59 g of (C₄H₉)₄NBH₃CN is added during about 2 hours, keeping the temperature below 30°C. The mixture is stirred under nitrogen for about 20 hours, then is acidified to pH about 1 with concentrated HCl and it is evaporated to dryness. The residue is taken up into 250 ml of absolute ethanol, 2,750 ml of ethyl ether are added, the obtained solid is filtered, taken up into water, adjusted to slightly basic pH (about 9.5) and extracted repeatedly with chloroform. The chloroform solutions are combined, washed with water to neutrality, dried over Na₂SO₄ and evaporated to dryness. The residue is taken up into 500 ml of methanol, saturated with HCl, decolorized with active charcoal, filtered, evaporated to dryness, taken up into 50 ml of absolute ethanol and precipitated with anhydrous ethyl ether. The precipitate is filtered and dried under vacuum at 40°C. 14.5 g are obtained of a mixture of the four stereoisomers (+) (αS, βR, γR); (-) (αR, βS, γS); (+) (αR, βS, γR); (-) (αS, βR, γS). (30% yield); m.p. 202-210°C: MF = C₂₁H₂₇NO₄.HCl MW = 393.92.

Elementary analysis, IR and NMR spectra are in agreement.

### EXAMPLE 2

### (αS, βR)-5,6-dimethoxy-2[[2(4-hydroxyphenyl)-2-hydroxy-1-methylethyl]amino]1,2,3,4-tetrahydronaphthalene hydrochloride (Ib).

A solution of 31.72 g (0.154 mol.) of 5,6-dimethoxy-2-tetralone in 428 ml of absolute ethanol, kept at a temperature from 10°C to 15°C, under nitrogen atmosphere, is slowly added with a solution of 13.99 g (0.084 mole) of (-) (αS, βR) 4-hydroxynorephedrine in 223 ml of absolute ethanol and glacial acetic acid to pH about 7.8. The mixture is stirred under nitrogen atmosphere for about 2 hours at room temperature. 4.51 g of 10% Pd/C are added and the mixture is hydrogenated with Parr system at room temperature at 30 p.s.i. for 20 hours. The reaction mixture is filtered, acidified to pH 1 with HCl and evaporated to dryness. The residue is taken up into 2,000 ml of water, washed with CHCl₃. The aqueous phase is adjusted to pH 9.2 with 10% KOH and repeatedly extracted with CHCl₃. The combined organic phases are washed with water to neutrality, dried over Na₂SO₄ and evaporated to dryness. The residue is taken up into methanol and decolorized with active charcoal, then filtered and evaporated under vacuum. The resulting solid is taken up into about 100 ml of absolute ethanol and the product is precipitated by addition of about 250 ml of anhydrous ethyl ether, then crystallized from 50:50 absolute ethanol/ethyl ether. 14.18 g of a mixture of the two diastereoisomers (-) (αS, βR, γS) and (+) (αS, βR, γR) are obtained (43% yield); m.p. = 214-218°C. MF = C₂₁H₂₇NO₄.HCl. MW = 393.92.
[α]²⁰ = (C = 1,87% in MeOH) -18,64°.

Elementary analysis, NMR and IR spectra in agreement.

Following the procedure described above but starting from 5,6-dimethoxy-2-tetralone and (+) ( R, βS) -4-hydroxynorephedrine, the following compound is obtained:
(αR, βS)-5,6-dimethoxy-2[[2-(4-hydroxyphenyl)-2-hydroxy-1-methylethyl]amino]1,2,3,4-tetrahydronaphthalene hydrochloride (Ic), consisting of a mixture of diastereoisomers (+) (αR, βS, γR) and (-) (αR, βS, γS)
m.p. = 219-221°C
MF = C₂₁H₂₇NO₄ _{.} HCl MW = 393,92
[α]²⁰ = (C = 2,002% in MeOH) -17,48°.

### EXAMPLE 3

### Erythro-5,6-dimethoxy-2[[2-(4-hydroxyphenyl)-2-hydroxy-1-methylethyl]amino]1,2,3,4-tetrahydronaphthalene hydrochloride (Ia).

a) 5,6-dimethoxy-2[[2-(4-benzyloxyphenyl)-2-oxo-1-methylethyl]amino]-1,2,3,4-tetrahydronaphthalene hydrochloride.
   A solution containing 20.0 g (0.082 mol.) of 5,6-dimethoxy-2-aminotetrahydronaphthalene (IV; R₁=H), 26.2 g (0.082 mol.) of 2-bromo(4 benzyloxy)-1-propiophenone (V) and 9.1 g (0.09 mol.) of triethylamine in 82 ml of 95% absolute ethanol is refluxed with stirring, under nitrogen atmosphere, for 6 hours. At the end of the reaction, the solution is evaporated under vacuum, the resulting residue is taken up into 500 ml of ethyl ether, under stirring. The resulting solid is filtered, taken up into 500 ml of a 15% K₂CO₃ aqueous solution and extracted with ethyl acetate ( 3 x 300 ml). The organic solution is washed with water and dried over Na₂SO₄, then filtered and added with ether/HCl, to precipitate the product as hydrochloride, which is filtered and recrystallized from 50:50 ethanol/ether. 28.8 g of a mixture of four stereoisomers of the following configurations: (+) (αR, γR); (-) (αS, γS); (-) (αR, γS); (+) (αS, γR) are obtained (73% yield). MW = 482.02 MF = C₂₈H₃₁NO₄ . HCl m.p. = 270-275°C.
   Elementary analysis and IR spectrum are in agreement.
b) Erythro-5,6-dimethoxy-2[[2-(4-hydroxyphenyl)-2-hydroxy-1-methylethyl]amino]1,2,3,4-tetrahydronaphthalene hydrochloride (Ia).
   3 g of 10% Pd/C are added to a solution containing 11.0 g (0.023 mol.) of 5,6-dimethoxy-2[[2-(4-benzyloxyphenyl)-2-oxo-1-methylethyl]amino]-1,2,3,4-tetrahydronaphthalene hydrochloride (VI; R₁=H, R₂=CH₂C₆H₅) in 600 ml of 95% CH₃OH and hydrogenation is carried out in Parr apparatus at room temperature and under 35 p.s.i. pressure for 30 minutes. The mixture is filtered, evaporated to dryness, triturated thoroughly in ethyl acetate, then the resulting solid is filtered, dried under vacuum, recrystallized from 50:50 ethanol/ether, to obtain 5.6 g of the product (81% yield)consisting of the same stereoisomers of Example 1.

### EXAMPLE 4

### Threo-5,6-dimethoxy-2[[2-(4-hydroxyphenyl)-2-hydroxy-1-methylethyl]amino]-1,2,3,4,-tetrahydronaphthalene hydrochloride (Id).

a) 5,6-dimethoxy-2[[2-(4-benzyloxyphenyl)-2-oxo-1-methylethyl]-N-benzylamino]-1,2,3,4-tetrahydronaphthalene (VI; R₁, R₂= CH₂-C₆H₅).
   32.1 g of 2-bromo(4-benzyloxy)-1-propiophenone (V) and 14 ml of triethylamine are added to a solution of 29.7 g of 5,6-dimethoxy-2-benzylamino-1,2,3,4-tetrahydronaphthalene (IV; R₁=CH₂-C₆H₅) in 90 ml of absolute ethanol. The reaction mixture is refluxed for 8 hours, slowly adding further 20 ml of triethylamine, then it is cooled and filtered washing the residue with ethanol and ether. The solution is evaporated to dryness, then adjusted to pH 8.2 with methanolic potassium hydroxide, evaporated to dryness, taken up into 200 ml of chloroform, washed with water and dried over Na₂SO₄, filtered and evaporated to dryness to obtain 30 g of the product which is further purified by chromatography (eluent : 70:30 hexane/ethyl acetate).
   23 g of the product are obtained (40% yield).
b) Threo-5,6-dimethoxy-2[[2-(4-benzyloxyphenyl)-2-hydroxy-1-methyl-ethyl]-N-benzylamino]-1,2,3,4-tetrahydronaphthalene hydrochloride (VII; R₁, R₂=CH₂-C₆H₅).
   A solution of 3.8 g 5,6-dimethoxy-2[[2-(4-benzyloxyphenyl)-2-oxo-1-methylethyl]-N-benzylamino]-1,2,3,4-tetrahydronaphthalene (VI; R₁, R₂=CH₂-C₆H₅) in 120 ml of tetrahydrofuran are added to a suspension of 0.32 g of lithium aluminium hydride in 60 ml of tetrahydrofuran, with stirring and under nitrogen atmosphere, at a temperature lower than 25°C. The reaction mixture is stirred at room temperature for 30 minutes, then 1 ml of ethyl acetate is added, stirring for 5 minutes, then the mixture is filtered through Celite ^{R} and ether/HCl (about 20 ml) is added to the clear solution to obtain a precipitate which is filtered and dried at 50°C under reduced pressure. 3.7 g of the product are obtained (97% yield).
c) Threo-5,6-dimethoxy-2[[2-(4-hydroxyphenyl)-2-hydroxy-1-methylethyl]amino]-1,2,3,4-tetrahydronaphthalene hydrochloride (Ic).
   2.3 g of 20% Pd/C are added to a solution containing 6,7 g di threo-5,6-dimethoxy-2[N[2-(4-benzyloxyphenyl)-2-hydroxy-1-methylethyl]-N-benzylamino]-1,2,3,4-tetrahydronaphthalene hydrochloride (VII; R₁, R₂=CH₂-C₆H₅) in 200 ml of absolute ethanol, then the mixture is hydrogenated in a Parr apparatus at room temperature under 35 p.s.i. pressure for 6 hours. The mixture is filtered, concentrated to about 60 ml, then ethyl ether (about 400 ml) is added thereto to complete precipitation. The resulting solid is recrystallized from acetone. 3.2 g of a mixture of four stereoisomers of the following configurations: (αS, βS, γS); (αR, βR, γR); (αS, βS, γR), (αR, βR, γS) are obtained (68% yield) m.p. = 200-201°C.

Elementary analysis, IR and NMR spectra are in agreement.

### EXAMPLE 5

(-) (αR, βS, γS)-5,6-dimethoxy-2-[[2-(4-hydroxyphenyl)-2-hydroxy-1-methylethyl]amino]-1,2,3,4-tetrahydronaphthalene hydrochloride (Ie).

1.5 g of compound Ic, consisting of a mixture of the two diastereoisomers (-) (αR, βS, γS) and (+) (αR, βS, γR), are dissolved in methanol. The solvent is evaporated to give a solid foam which is taken up with 15 ml of methanol and the obtained suspension is refluxed for 4 hours. The mixture is cooled to 60°C, the solid is filtered, washed with diethyl ether and dried under vacuum at 60°C.

0.5 g a white crystalline solid are obtained.
[α]²⁰ = -40.27° (C=1.008 in MeOH)
[α]²⁰ = -48.21° (C=1.008 in MeOH)
- G.L.C. analysis:: % of (-) (αR, βS, γS) = 93.5
% of (+) (αR, βS, γR) = 6.5.

In a similar way, starting from a mixture of the two stereoisomers (+) (αS, βR, γR) and (-) (αS, βR, γS), (+) (αS, βR, γR))-5,6-dimethoxy-2-[[2-(4-hydroxyphenyl)-2hydroxy-1-methylethyl]amino]-1,2,3,4-tetrahydronaphthalene hydrochloride is obtained
m.p. = 230°-231°C
[α]²⁰ = +35.85° (C=1.00 in MeOH)
[α]²⁰ = +43.32° (C=1.00 in MeOH)
- G.L.C. analysis:: % of (+) (αS, βR, γR) = 92.5
% of (-) (αS, βR, γS) = 7.5.

### EXAMPLE 6

(+) (αR, βS, γR)-5,6-dimethoxy-2-[[2-(4-hydroxyphenyl)-2-hydroxy-1-methylethyl]amino]-1,2,3,4-tetrahydronaphthalene hydrochloride.
a) (+) (αR, γR)-5,6-dimethoxy-2-[(2-(4-benzyloxyphenyl)-2-oxo-1-methylethyl]amino]-1,2,3,4-tetrahydronaphthalene hydrochloride.
   1.2 g of (+) (R)-5,6-dimethoxy-2-aminotetrahydronaphthalene and 1.9 g of 2-bromo-(4-benzyloxy)-1-propiophenone are dissolved in 11 ml of ethanol, added with 0.8 of triethylamine and refluxed for 4 hours.
   After standing overnight, the solid is filtered which consist of the diastereoisomer (+) (αS, γR). The filtrate is evaporated, the residue is dissolved in 5 ml of ethanol, 1.5 ml of a 10% triethylamine HCl in ethanol are added, the mixture is stirred for 1 hour, kept at 4°C for 2 days, the solid is filtered off, the filtrate is evaporated, the residue is dissolved in 30 ml of ethanol and, after addition of a 1 M HCl solution in methanol, the mixture is allowed to stand for 2 hours.
   The precipitated solid is filtered, washed with ethanol and then with ether and dried at 40°C under vacuum.
   The obtained solid is suspended in 25 ml of a refluxing 6:4 ethanol/methanol mixture for 1 hour. After standing 2 hours, the mixture is filtered, washed with diethyl ether and dried under vacuum at 40°C.
   0.4 g a white solid consisting of the stereoisomer (+) (αR, γR) are obtained with a diastereoisomeric purity ≃ 90%
   m.p. = 263-265°C (dec.)
b) (+) (αR, βS, γR)-5,6-dimethoxy-2-[[2-(4-hydroxyphenyl)-2-hydroxy-1-methylethyl]amino]-1,2,3,4-tetrahydronaphthalene hydrochloride.

0.4 g of (+) (αR, γR)-5,6-dimethoxy-2-[[2-(4-benzyloxyphenyl)-2-oxo-1-methylethyl]amino]-1,2,3,4-tetrahydronaphthalene hydrochloride are suspended in 25 ml of methanol. 0.1 g of 5% Pd/C are added and the the mixture is hydrogenated at 35 p.s.i. under stirring.

The catalyst is filtered, the solvent is evaporated and the solid foam is dissolved in 10 ml of warm acetone, filtering off the solid residue and cooling to room temperature under stirring.

After standing overnight, the solid is filtered, washed with diethyl ether and dried at 70°C under vacuum for 24 hours.

0.2 g of (+) (αR, βS, γR) isomer are obtained
[α]²⁰ = +51.45° (C=1.036 in MeOH)
[α]²⁰ = +60.91° (C=1.036 in MeOH)
- G.L.C. analysis:: % of (+) (αR, βS, γR) = 98.49
% of (+) (αS, βR, γR) = 1.51

With the same method, starting from
(-) (S)-5,6-dimethoxy-2-aminotetrahydronaphthalene and 2-bromo-(4-benzyloxy)-1-propiophenone, the following compounds are obtained:
a) (-) (αS, γS)-5,6-dimethoxy-2-[[2-(4-benzyloxyphenyl)-2-oxo-1-methylethyl]amino]-1,2,3,4-tetrahydronaphtalene hydrochloride.
b) (-) (αS, βR, γS)-5,6-dimethoxy-2-[[2-(4-hydroxyphenyl)-2-hydroxy-1-methylethyl]amino]-1,2,3,4-tetrahydronaphtalene hydrochloride (Ih).

[α]²⁰ = -49.03° (C=1.03 in MeOH)
- G.L.C. analysis:: % of (-) (αS, βR, γS) = 95.9
% of (-) (αR, βS, γS) = 4.1.

The inotropic and chronotropic effects of compound (Ia) of the invention were determined in vitro using, respectively, isolated rat left atrium and right atrium.

### Left atrium : inotropic activity.

The atria were subjected to a 0.5 g starting tension and suspended in a bath containing aerated Krebs-Henseleit solution with 95% O₂ and 5% CO₂ kept at 32°C. The preparation was stimulated at a 4 Hz frequency with pulses during 5 msec each. The contraction strength was detected by an isometric transducer and recorded on a microdynamometer.

### Right atrium : chronotropic activity.

The preparation was prepared in the same way and under the same conditions as described above, but it was not subjected to electric stimulation. Frequency was detected by a cardiotachograph.

The results were expressed as the percentage of the response obtained with a maximal dose (10⁻⁸ M) of isoproterenol which was determined at the beginning of the test. In Table 1 the potency (EC₅₀ = effective concentration inducing the 50% of the maximal effect) and the relative intrinsic activity (expressed as α and compared with that of isoproterenol, taken = 1) are shown.

Beside compound Ia and the control compound, two other 2-aminotetralin derivatives, i.e. compounds (VIII) and (IX), were assayed in these tests. Said compounds were prepared within the scope of a structure-activity research carried out in order to define the structural characteristics necessary to cause the desired activity in this kind of compounds.

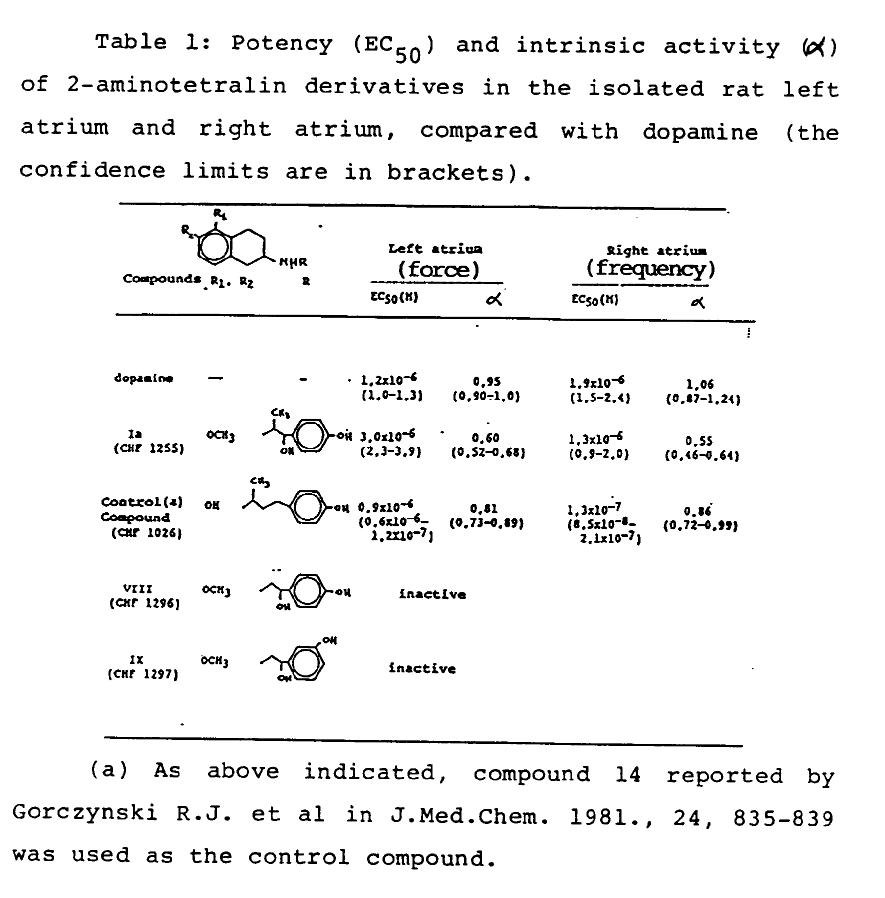

For the sake of shortness, hereinafter the compounds will be identified by means of the experimental abbrevations (CHF) shown in the Table.

As it can be desumed by the data reported in Table 1, CHF 1255 proved to exert in these preparations a cardiostimulating activity with a potency comparable to those of dopamine and of CHF 1026, which was used as the prior art reference compound.

However, contrary to the control compounds, CHF 1255 shows a reduced intrinsic activity : therefore, based on its activity, which, as will be evidenced hereinafter, is of the beta-1 stimulating kind, it must be considered as a partial agonist of beta-1 adrenergic receptors. This behaviour was further confirmed in isolated left atria of other animal species (cat and guinea pig), in which the compound shows an inotropic activity with an effectiveness of 45-31% that of isoproterenol. The partial agonism of CHF 1255 towards beta-1 adrenergic receptors is of great importance for the characterization of the compound.

In fact, the research efforts have been directed for a long time to the synthesis and development of partial agonistic beta-1 stimulating molecules : starting from this assumption, prenalterol and, more recently, xamoterol, have been prepared, the latter being less effective ( it has an agonistic action of 40% that of isoproterenol), but better tolerated.

The rational of the therapy with partial agonists is related to the induction of a beta-agonistic effect that can be modulated. As a matter of fact these compounds, acting both as agonists and as antagonists, stimulate the impaired cardiac function in patients affected with cardiac failure, under rest or reduced exercise conditions.

On the contrary, during physical exercise, when the sympatethic tonus is high, they are able to protect the heart against adrenergic overstimulation, antagonizing its effects.

Another important fact evident from Table 1 is that CHF 1296 and CHF 1297 proved to be completely inactive, further confirming thereby that minimal structural changes play in this kind of compounds a fundamental role for the activity.

CHF 1255 has been subsequently subjected to a series of tests in order to study the mechanism of its inotropic activity.

It has been possible to show, as previously said, that this activity is due to the stimulation of beta-1 adrenergic receptors.

Studies carried out in different experimental models allowed to exclude that the cardiostimulant action of the compound may be due to catecholamines release or to the stimulation of alpha-adrenergic cardiac receptors as well as to the stimulation of histaminergic H₁ and H₂ receptors.

It has also been excluded that the cardiostimulant activity of CHF 1255 is due to phosphodiesterase inhibition.

### Isolated rat left atrium: interactions with beta - adrenergic receptors.

The affinity for beta-1 adrenergic receptors has been evaluated in the rat left atrium as previously described.

In this test, dose - response curves of CHF 1255 were recorded in the absence and in the presence of increasing concentrations (10⁻⁸ - 3 x 10⁻⁷ M) of a selective beta-1 blocker, atenolol.

The pre-treatment with the beta-1 blocker shifted to the right in a dose-dependent way the dose - response curve of CHF 1255, showing therefore for this compound an interaction of competitive type for the beta-1 adrenergic receptors.

The selectivity of this interaction has been studied by evaluating the effects on beta-2 adrenergic receptors, in the guinea-pig isolated trachea, using CHF 1026 as reference compound.

### Isolated guinea-pig trachea: interactions with beta-2 adrenergic receptors.

Trachea strips were isolated and prepared according to the method described by Emerson J e Mackay D in J Pharm Pharmacol 1979, 31, 798, immersed in Krebs-Henseleit solution (37°C, 95% 0₂ + 5% C0₂), subjected to an initial tension of 1 g and contracted with carbachol (3 x 10⁻⁷ M).

Dose-response curves of CHF 1255 and CHF 1026 were recorded in the absence and in the presence of propranolol (10⁻⁶ M), used as antagonist and added to the bath before the stimulation with carbachol.

The obtained results are shown in Table 2, expressed as potency (EC₅₀ calculated from the dose - response curve) and intrinsic activity (α, isoproterenol=1).

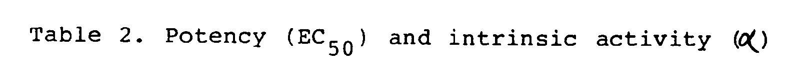

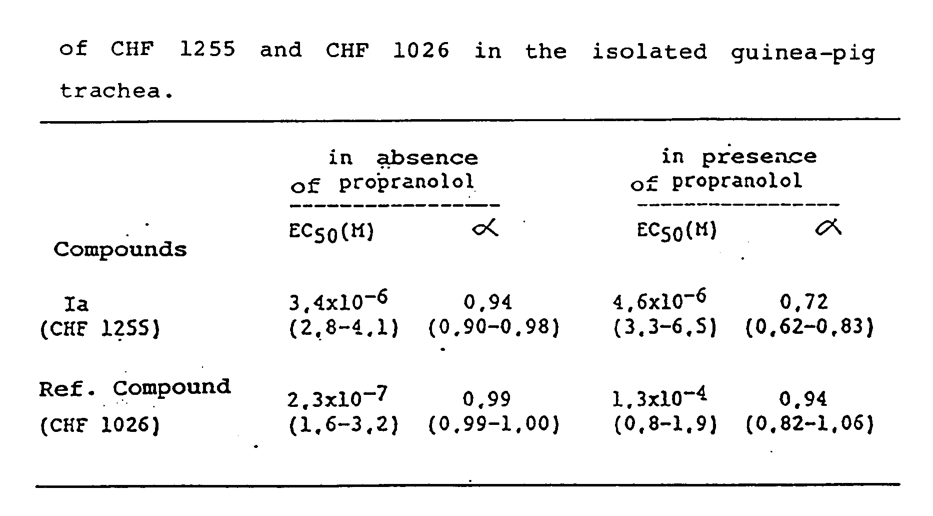

Both compounds exert a dose-dependent inhibition of the carbachol-induced contractions, but while in the presence of propranolol the dose-response curve of CHF 1026 is parallelely shifted to the right, showing a competitive antagonistic action, the effects of CHF 1255 remain unchanged.

According to these results it is possible to state that, differently from CHF 1026, CHF 1255 does not stimulate beta-2 adrenergic receptors. The relaxing effect is probably to be ascribed to other mechanisms, since it is not competitively antagonized by an high dose of propranolol.

Because of its selective agonistic activity on adrenergic beta-1 receptors, CHF 1255, differently from CHF 1026, has no tachycardic and arrhythmogenic activity due to the stimulation of beta-2 receptors.

The activity of CHF 1255 has been compared with two recently introduced inotropic drugs, having different mechanisms of action, xamoterol fumarate and amrinone, the former being a partial agonist of adrenergic beta-1 receptors and the latter being an inhibitor of phosphodiesterase.

### Comparison of inotropic activity of CHF 1255 with xamoterol and amrinone.

The effects of the compounds were evaluated in rat isolated atria according to the previously described method.

The results are reported in Table 3.

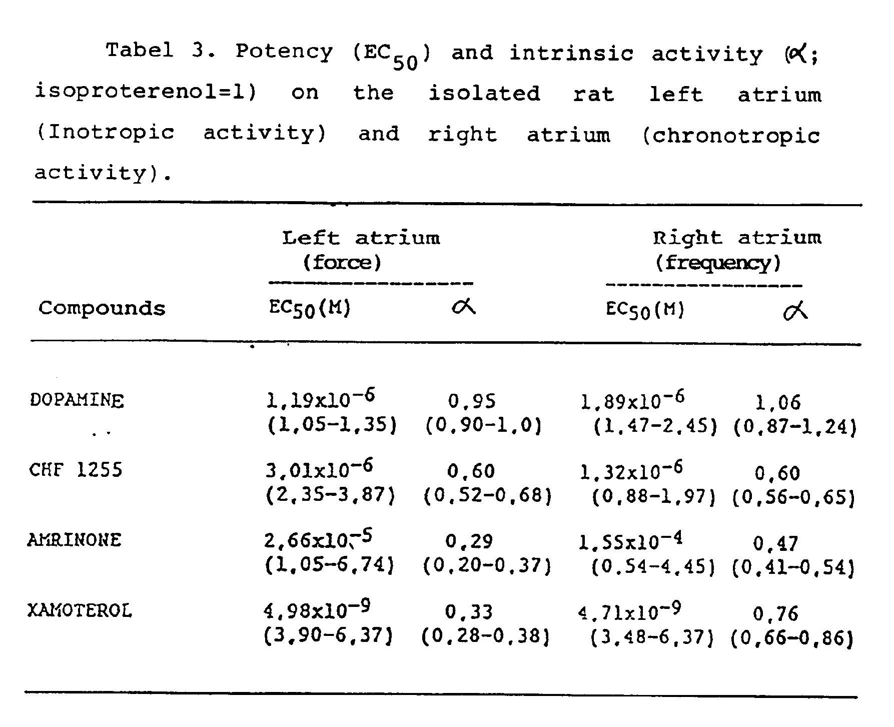

The potency of CHF 1255, exspressed as concentration producing 50% of the maximum effect (increase of contraction force), is about 10 times higher than that of amrinone and about 300 times lower than that of xamoterol.

It should be noticed that the chronotropic efficacy of CHF 1255 is remarkably lower than that of xamoterol.

This is very important because an inotropic drug should not cause an excessive increase of heart-rate.

CHF 1255 also induces an important hemodynamic action, differently from xamoterol.

### Rat perfused hind quarters: vasodilating action.

The vasodilating effect of CHF 1255 has been evaluated in the arterial rat hind quarter perfused with Krebs-Henseleit solution at constant flow.

The increase of arterial resistances was induced by K⁺-enriched solution (40 mM), norepinephrine (5 x 10⁻⁶ M) and serotonin (5 x 10⁻⁷ M).

Papaverine and prazosin, powerful α-blocker vasodilator, were used as reference compounds.

The results are reported in Table 4.

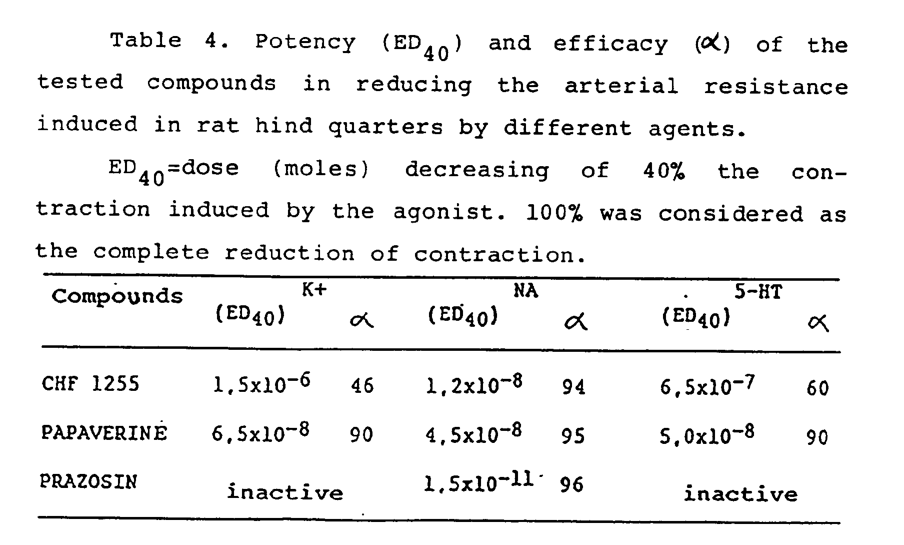

CHF 1255 is active against the different agents but, differently from papaverine having the same ED₄₀ for the different experimental conditions, proved to be particularly active on the norepinephrine induced contraction.

This effect is due to an α-blocking activity whereas the activity against the potassium or serotonin induced contraction is probably due to an aspecific mechanism which could even account for the relaxing activity on the isolated guinea-pig trachea which is not mediated by beta-2 receptors.

The α-blocking activity of CHF 1255 has been confirmed in the rabbit isolated aorta, with a competitive inhibition of the norepinephrine dose - response curve.

The interesting hemodynamic properties of CHF 1255 were confirmed in vivo in the anaesthetized dog.

### Anaesthezized dog: cardiovascular activity.

Beagle dogs were anaesthetized with sodium penthobarbital (30 mg/kg iv) thoracotomized and instrumented for recording cardiocirculatory parameters.

CHF 1255 and the reference drugs (amrinone and xamoterol) were administered i.v. in bolus.

CHF 1255 at the doses of 0.3-1-3 µmoles/kg i.v. confirmed its activity in changing hemodynamic parameters.

It has in fact, differently from xamoterol, whose effects are negligible, a good hypotensive action (respectively -17, -22 and -31%) and a marked reduction of the systemic arterial resistances (-28, -37 and -45%) together with a significant increase of the stroke volume (10, 16 and 21) and of the cardiac output (17,28 and 34%).

It shoud be noted that the hemodynamic effects are more relevant than those of amrinone (1-3-10 µmol/kg iv).

CHF 1255 showed also remarkable inotropic effect increasing the dF/dT index (peak of the first derivative of the aortic flow in ml/sec⁻²), useful for the evaluation of the cardiac function.

CHF 1255 has shown therefore to have a very interesting pharmacological profile and it has to be considered as belonging to the class of "inodilators" since it is endowed with both inotropic and vasodilator actions.

Its activity is also long-lasting probably because not easily subjected to metabolic inactivation by catechol-0-methyltransferases (COMT), because of the methylation of the catecholic groups.

We have also studied wether the pharmacodynamic effects of CHF 1255 were to be ascribed to a particular isomer, by carring out tests using the diastereoisomeric mixture (αS, βR) (CHF 1389) and the diastereoisomeric mixture (αR, βS) (CHF 1388).

CHF 1389 proved to be as effective as CHF 1255 in the rat isolated left atrium whereas CHF 1388 did not show any inotropic activity.

On the other hand, the vasodilating activity of CHF 1255 is mainly due to CHF 1388.

The overall activity of CHF 1255 (Compound Ia) is therefore due to a synergic interaction of the effects produced by the two diastereoisomeric mixtures, each having its specific action so as to justify an independent therapeutic use.

### Evaluation of acute toxicities.

The results are shown in Table 5.

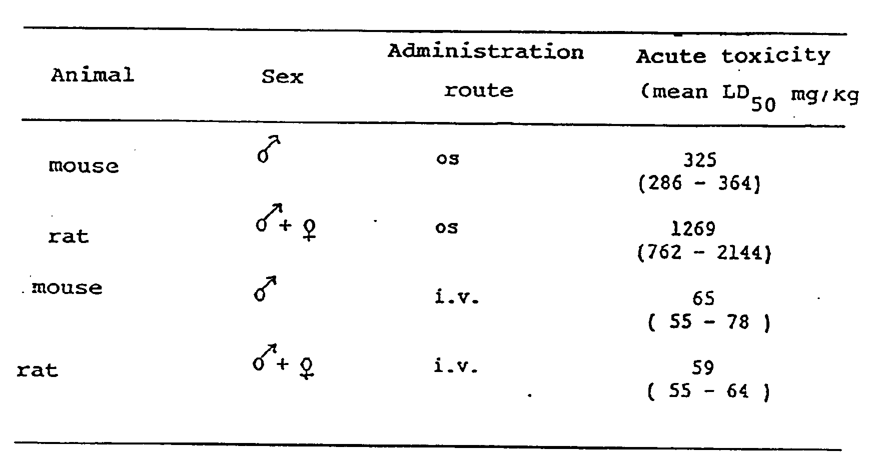

The present invention also relates to pharmaceutical compositions containing as the active ingredient the compound of formula (I) or one of the isomers thereof as such or in form of a pharmaceutically acceptable salt thereof, in combination with pharmaceutically acceptable excipients. Said compositions can be used for the cardiovascular therapy, particularly as inotropic agents.

The compositions will be administered preferably by the oral or parenteral routes, in form of capsules, tablets or vials respectively.

The pharmaceutical compositions for the oral administration in unit doses, can be prepared by admixing the active ingredient with a solid powdered excipient, such as lactose, saccharose, sorbitol, mannitol; potato, cereal or maize starch, amylopectin, cellulose or gelatin derivatives, and optionally also with lubricants such as talc, magnesium or calcium stearate, polyethylene glycol or silica.

Tablets can be variously coated according to well-known pharmaceutical techniques. Hard gelatin capsules can contain granulates of the active ingredient, together with solid powdered excipients, such as lactose, saccharose, sorbitol, mannitol, starches of the above indicated types, cellulose or gelatin derivatives, and they can also contain stearic acid or magnesium stearate or talc. The unit dose for the above described formulations will range from 10 to 100 mg of the active ingredient.

In case of injectable formulations for the parenteral administration, the excipients can be a pharmaceutically acceptable sterile liquid, such as water or an aqueous polyvinylpyrrolidone solution and optionally a stabilizer and/or a buffer.

The active ingredient can be dissolved in the liquid and filter sterilized before the distribution into vials, or it can be directly freeze-dried, in which case vials containing injection liquid will be contained in the packages, to restore the solution before use.

Another particularly advantageous methods for the administration of the compounds of the invention is provided by transdermic systems, which consist of adhesive matrices which can be applied to skin. In said systems the active ingredient is incorporated in an appropriate concentration and is gradually released through the skin to enter the hematic circle.

## Claims (Claims for the following Contracting State(s): DE, GB, FR, IT, NL, SE, LI, CH, BE, AT, LU, DK)

1. The compound of formula 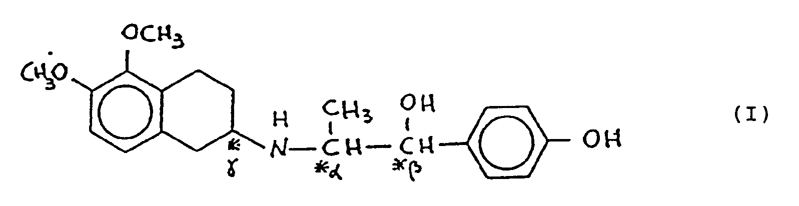 the stereoisomers thereof and the mixtures of one or more stereoisomers and the pharmaceutically acceptable salts thereof.

2. A compound of claim 1 selected in the group of:
erythro-5,6-dimethoxy-2[[2-(4-hydroxyphenyl)-2-hydroxy-1-methylethyl]amino]-1,2,3,4-tetraydronaphtalene;
threo-5,6-dimethoxy-2[[2-(4-hydroxyphenyl)-2-hydroxy-1-methylethyl]amino]-1,2,3,4-tetraydronaphtalene;
( R, βS)-5,6-dimethoxy-2[[2-(4-hydroxyphenyl)-2-hydroxy-1-methylethyl]amino]-1,2,3,4-tetraydronaphtalene;
( S, βR) 5,6-dimethoxy-2[[2-(4-hydroxyphenyl)-2-hydroxy-1-methylethyl]amino]-1,2,3,4-tetraydronaphtalene;
(-) ( R, βS, S)-5,6-dimethoxy-2[[2-(4-hydroxyphenyl)-2-hydroxy-1-methylethyl]amino]-1,2,3,4-tetraydronaphtalene;
(+)( S, βR, R)-5,6-dimethoxy-2[[2-(4-hydroxyphenyl)-2-hydroxy-1-methylethyl]amino]-1,2,3,4-tetraydronaphtalene;
(+) ( R, βS, R)-5,6-dimethoxy-2[[2-(4-hydroxyphenyl)-2-hydroxy-1-methylethyl]amino]-1,2,3,4-tetraydronaphtalene;
(-) ( S, βR, S)-5,6-dimethoxy-2[[2-(4-hydroxyphenyl)-2-hydroxy -1-methylethyl]amino]-1,2,3,4-tetraydronaphtalene.

3. A process for the preparation of the compound of claim 1, in which 5,6-dimethoxy-2-tetralone (II) 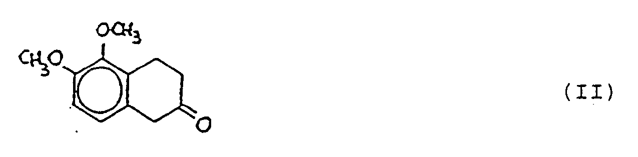 is subjected to reductive amination with the amine of formula (III)

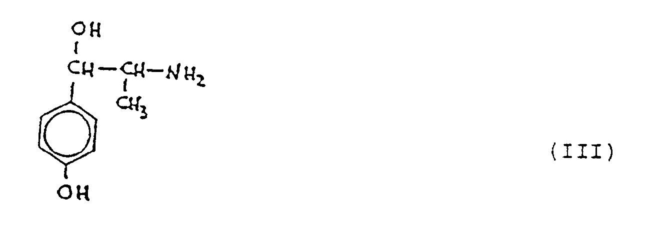

4. The process as claimed in claim 3, in which the reductive amination is carried out using metal hydrides.

5. The process as claimed in claim 4, in which sodium cyanoborohydride or a mixture or sodium cyanoborohydride with tetrabutylammonium cyanoborohydride in a 4:1 to 1:1 weight ratio are used as the metal hydrides.

6. The process for the preparation of the compounds as claimed in claim 1, which comprises subjecting to reduction a compound of formula (VI) 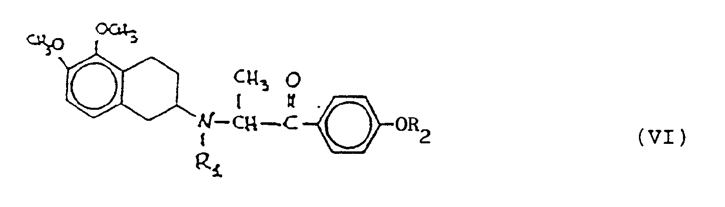 wherein R₁ and R₂ are hydrogen or a benzyl group or a group having similar steric hindrance, which groups can subsequently be removed with per se known methods.

7. Compounds of formula (VI) 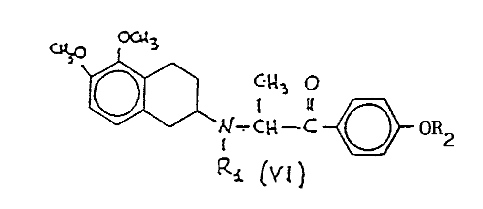 wherein R₁ and R₂ are hydrogen or a benzyl group or a group having similar steric hindrance, as intermediates for the preparation of the compounds of claim 1.

8. Pharmaceutical compositions for the treatment of cardiovascular diseases such as cardiac failure, containing as the active ingredient a compound as claimed in claims 1-2, in amounts from 10 to 100 mg per unit dose.

9. The use of a compound as claimed in claims 1-2 for the preparation of a medicament having cardiovascular activity.

## Claims (Claims for the following Contracting State(s): ES, GR)

1. A process for the preparation of the compound of formula (I) 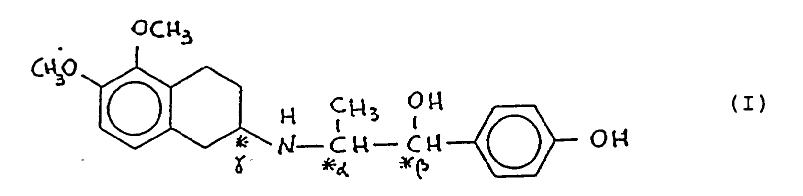 in which 5,6-dimethoxy-2-tetralone (II) 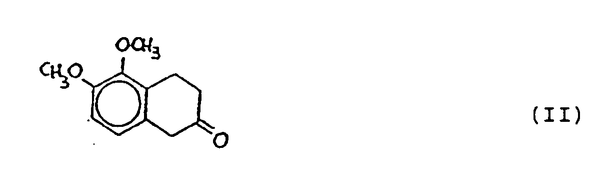 is subjected to reductive amination with the amine of formula (III)

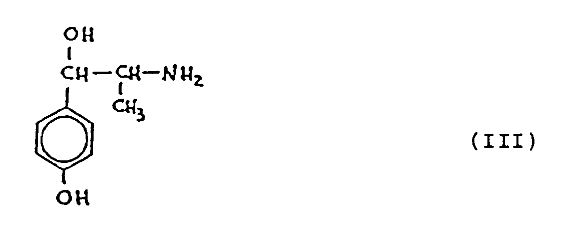

2. The process as claimed in claim 1, in which the reductive amination is carried out using metal hydrides.

3. The process as claimed in claim 2, in which sodium cyanoborohydride or a mixture or sodium cyanoborohydride with tetrabutylammonium cyanoborohydride in a 4:1 to 1:1 weight ratio are used as the metal hydrides.

4. The process for the preparation of the compounds of formula (I) 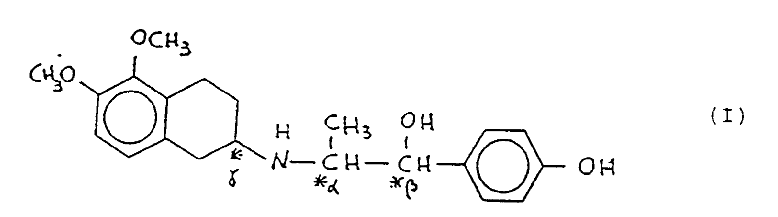 which comprises subjecting to reduction a compound of formula (VI) 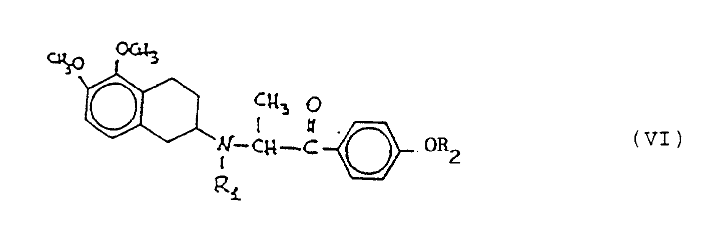 wherein R₁ and R₂ are hydrogen or a benzyl group or a group having similar steric hindrance, which groups can subsequently be removed with per se known methods.

## Patentansprüche (Patentansprüche für folgende(n) Vertragsstaat(en): DE, GB, FR, IT, NL, SE, LI, CH, BE, AT, LU, DK)

1. Verbindung der Formel 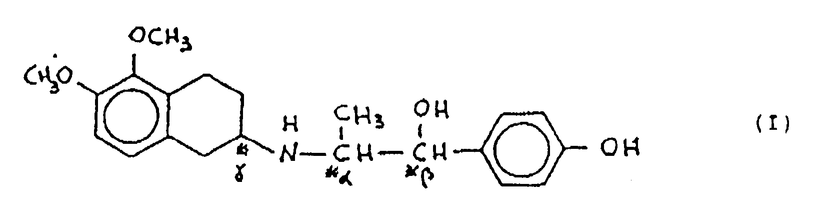 die Stereoisomere hiervon und die Mischungen von einem oder mehreren Stereoisomeren und die pharmazeutisch verwertbaren Salze hiervon.

2. Verbindung nach Anspruch 1, ausgewählt aus der Gruppe von: Erythro-5,6-dimethoxy-2[[2-(4-hydroxyphenyl)-2-hydroxy-1-methylethyl]amino]-1,2,3,4-tetrahydronaphtalin, Threo-5,6-dimethoxy-2[[2-(4-hydroxyphenyl)-2-hydroxy-1-methylethyl]amino]-1,2,3,4-tetrahydronaphtalin; ( R, βS)-5,6-Dimethoxy-2[[2-(4-hydroxyphenyl)-2-hydroxy-1-methylethyl]amino]-1,2,3,4-tetrahydronaphtalin; ( S, βR) 5,6-Dimethoxy-2[[2-(4-hydroxyphenyl)-2-hydroxy-1-methylethyl]amino]-1,2,3,4-tetrahydronaphtalin; (-) ( R, βS, S)-5,6-Dimethoxy-2[[2-(4-hydroxyphenyl)-2-hydroxy-1-methylethyl]amino]-1,2,3,4,-tetrahydronaphtalin; (+) ( S, βR, R)-5,6-Dimethoxy-2[[2-(4-hydroxyphenyl)-2-hydroxy -1-methylethyl]amino]-1,2,3,4-tetrahydronaphtalin; (+) ( R, βS, R)-5,6-Dimethoxy-2[[2-(4-hydroxyphenyl)-2-hydroxy-1-methylethyl]amino]-1,2,3,4-tetrahydronaphtalin; (-) ( S, βR, S)-5,6-Dimethoxy-2[[2-(4-hydroxyphenyl)-2-hydroxy-1-methylethyl]amino]-1,2,3,4-tetrahydronaphtalin.

3. Verfahren zur Herstellung der Verbindung nach Anspruch 1, dadurch gekennzeichnet, daß 5,6-Dimethoxy-2-tetralon (II) 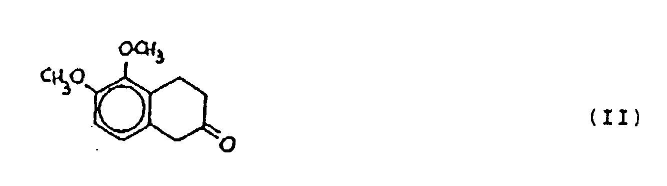 einer reduktiven Aminierung mit dem Amin der Formel (III) 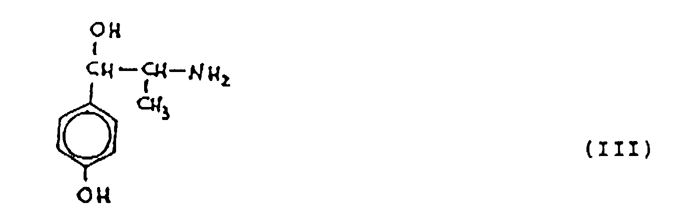 unterworfen wird.

4. Verfahren nach Anspruch 3, dadurch gekennzeichnet, daß die reduktive Aminierung unter Verwendung von Metallhydriden durchgeführt wird.

5. Verfahren nach Anspruch 4, dadurch gekennzeichnet, daß Natriumcyanoborhydrid oder eine Mischung von Natriumcyanoborhydrid mit Tetrabutylammonium-Cyanoborhydrid in einem 4:1 bis 1:1 Gewichtsverhältnis als Metallhydride verwendet werden.

6. Verfahren zur Herstellung der Verbindungen nach Anspruch 1, dadurch gekennzeichnet, daß eine Verbindung der Formel (VI) 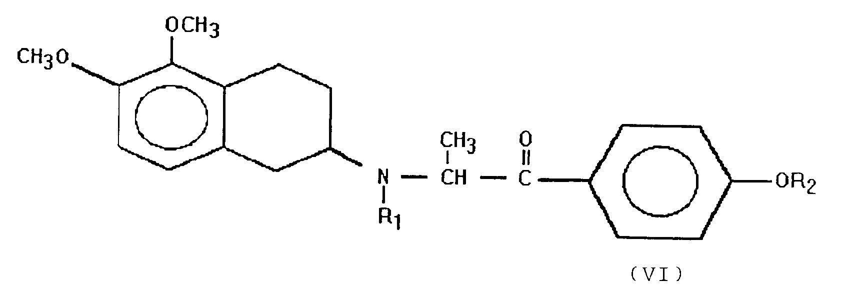 einer Reduktion unterworfen wird, worin R₁ und R₂ Wasserstoff oder eine Benzylgruppe oder eine Gruppe mit ähnlicher sterischer Hinderung sind, wobei diese Gruppen anschließend mit an sich bekannten Verfahren entfernt werden können.

7. Verbindung der Formel (VI) 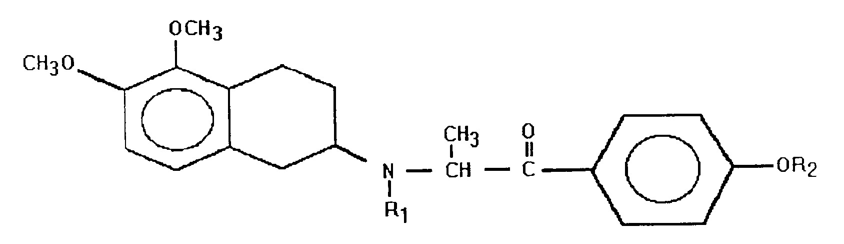 worin R₁ und R₂ Wasserstoff oder eine Benzylgruppe oder eine Gruppe mit ähnlicher sterischer Hinderung sind, als Zwischenprodukte zur Herstellung der Verbindungen des Anspruches 1.

8. Pharmazeutische Zusammensetzungen zur Behandlung von Kardiovaskulär-Krankheiten, solche wie Herzversagen, enthaltend als Wirkstoff eine Verbindung nach Anspruch 1 bis 2 in Mengen von 10 bis 100 mg pro Einheitsdosis.

9. Verwendung einer Verbindung nach Anspruch 1 bis 2 für die Herstellung eines Medikaments mit kardiovaskulärer Wirkung.

## Patentansprüche (Patentansprüche für folgende(n) Vertragsstaat(en): ES, GR)

1. Verfahren zur Herstellung der Verbindung gemäß Formel (I) 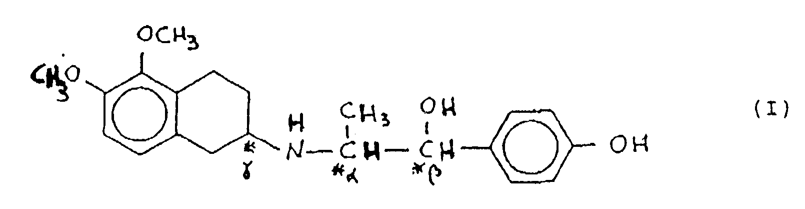 in der 5,6-Dimethoxy-2-tetralon (II) 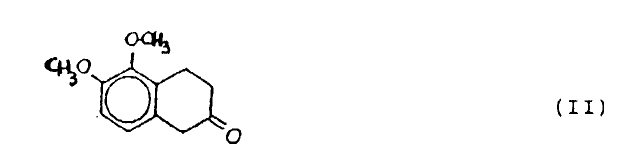 einer reduktiven Aminierung mit dem Amin der Formel (III) 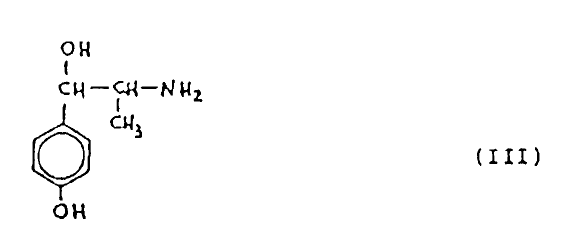 unterworfen wird.

2. Verfahren nach Anspruch 3, dadurch gekennzeichnet, daß die reduktive Aminierung unter Verwendung von Metallhydriden durchgeführt wird.

3. Verfahren nach Anspruch 4, dadurch gekennzeichnet, daß Natriumcyanoborhydrid oder eine Mischung von Natriumcyanoborhydrid mit Tetrabutylammonium-Cyanoborhydrid in einem 4:1 bis 1:1 Gewichtsverhältnis als Metallhydride verwendet werden.

4. Verfahren zur Herstellung der Verbindungen der Formel (I), 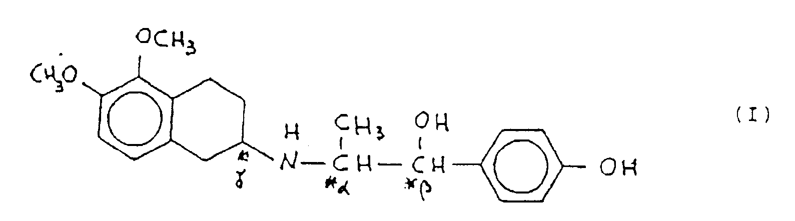 dadurch gekennzeichnet, daß eine Verbindung der Formel (VI) 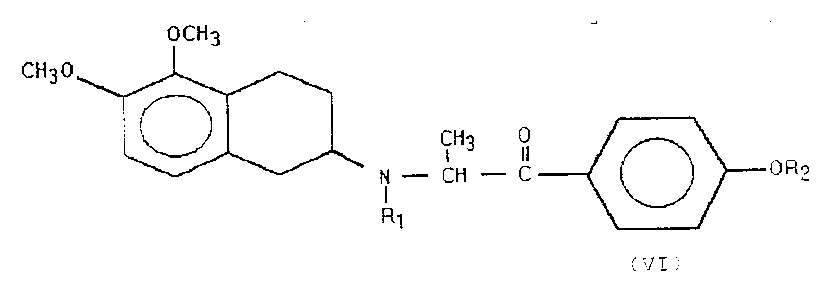 einer Reduktion unterworfen wird, worin R₁ und R₂ Wasserstoff oder eine Benzylgruppe oder eine Gruppe mit ähnlicher sterischer Hinderung sind, wobei diese Gruppen anschließend mit an sich bekannten Verfahren entfernt werden können.

## Revendications (Revendications pour l'(les) Etat(s) contractant(s) suivant(s): DE, GB, FR, IT, NL, SE, LI, CH, BE, AT, LU, DK)

1. Le composé de formule 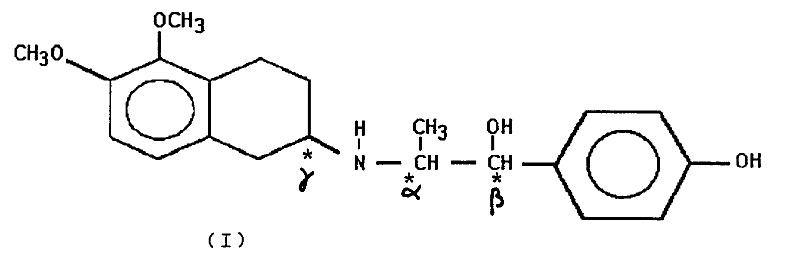 ses stéréoisomères et les mélanges d'un ou plusieurs stéréoisomères, et ses sels pharmaceutiquement acceptables.

2. Composé selon la revendication 1, choisi parmi : érythro-5,6-diméthoxy-2[[2-(4-hydroxyphényl)-2-hydroxy-1-méthyléthyl]amino]-1,2,3,4-tétrahydronaphtalène; thréo-5,6-diméthoxy-2[[2-(4-hydroxyphényl)-2-hydroxy-1-méthyléthyl]amino]-1,2,3,4-tétrahydronaphtalène; (R, BS)-5,6-diméthoxy-2[[2-(4-hydroxyphényl)-2-hydroxy-1(méthyléthyl]amino]-1,2,3,4-tétrahydronaphtalène; (S, BR)-5,6-diméthoxy-2[[2-(4-hydroxyphényl)-2-hydroxy-1-méthyléthyl]amino]-1,2,3,4-tétrahydronaphtalène; (-)(R, BS, S)-5,6-diméthoxy-2[[2-(4-hydroxyphényl)-2-hydroxy-1-méthyléthyl]amino]-1,2,3,4-tétrahydronaphtalène; (+)(S, BR, R)-5,6-diméthoxy-2[[2-(4-hydroxyphényl)-2-hydroxy-1-méthyléthyl]amino]-1,2,3,4-tétrahydronaphtalène; (+)(R, BS, R)-5,6-diméthoxy-2[[2-(4-hydroxyphényl)-2-hydroxy-1-méthyléthyl]amino]-1,2,3,4-tétrahydronaphtalène; (-)(S, BR, S)-5,6-diméthoxy-2[[2-(4-hydroxyphényl)-2-hydroxy-1-méthyléthyl]amino]-1,2,3,4-tétrahydronaphtalène.

3. Procédé de préparation du composé selon la revendication 1, dans lequel la 5,6-diméthoxy-2-tétralone (II) 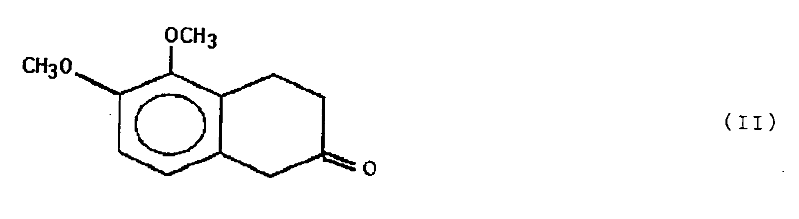 est soumise à une amination réductrice par l'amine de formule (III)

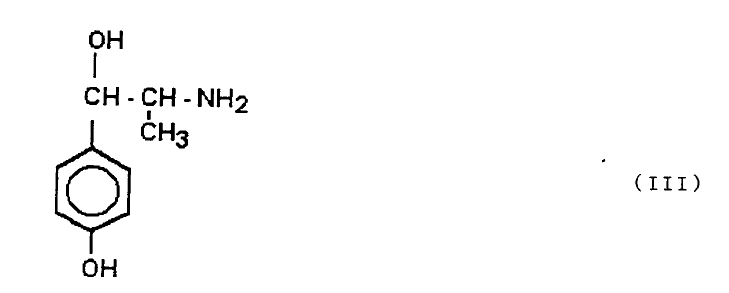

4. Le procédé selon la revendication 3, dans lequel l'amination réductrice est effectuée en utilisant des -hydrures métalliques.

5. Le procédé selon la revendication 4, dans lequel on utilise comme hydrures métalliques le cyanoborohydrure de sodium ou un mélange de cyanoborohydrure de sodium et de cyanoborohydrure de tètrabutylammonium dans un rapport en poids de 4:1 à 1:1.

6. Le procédé de préparation des composés revendiqués dans la revendication 1, qui comprend la réduction d'un composé de formule (VI) 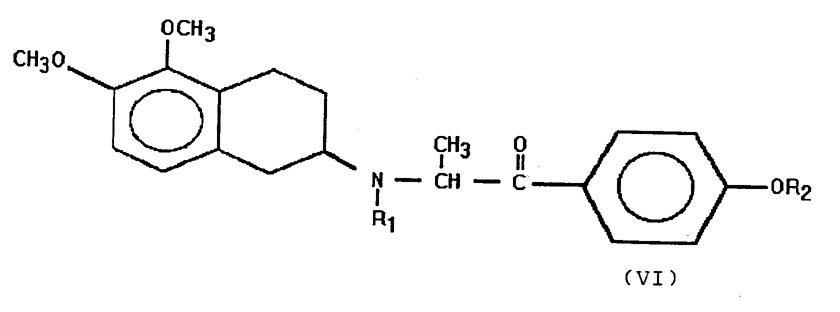 dans laquelle R₁ et R₂ sont l'hydrogène ou un groupe benzyle ou un groupe présentant un empêchement stérique similaire, groupe qui peut ensuite être éliminé par des méthodes connues en elles-mêmes.

7. Composés de formule (VI) 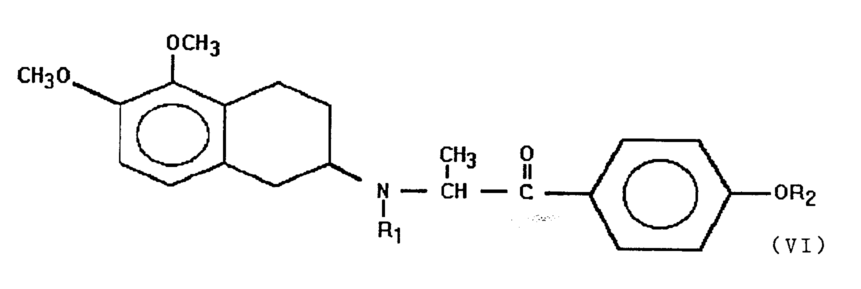 dans laquelle R₁ et R₂ sont l'hydrogène ou un groupe benzyle ou un groupe présentant un empêchement stérique similaire, comme intermédiaires de préparation des composés selon la revendication 1.

8. Compositions pharmaceutiques pour le traitement des affections cardiaques telles que l'insuffisance cardiaque, contenant comme ingrédient actif un composé tel que revendiqué dans les revendications 1-2, en quantités allant de 10 à 100 mg par dose unitaire.

9. L'utilisation d'un composé tel que revendiqué dans les revendications 1-2 pour la préparation d'un médicament ayant une activité cardiovasculaire.

## Revendications (Revendications pour l'(les) Etat(s) contractant(s) suivant(s): ES, GR)

1. Procédé de préparation du composé de formule (I) 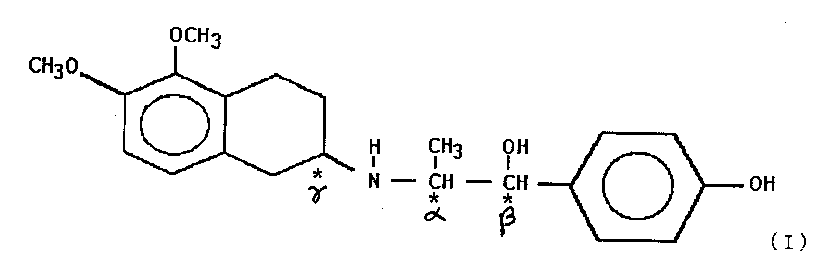 dans lequel la 5,6-diméthoxy-2-tétralone (II) 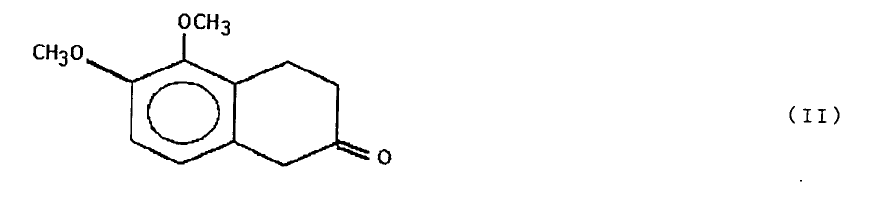 est soumise à une amination réductrice par l'amine de formule (III)

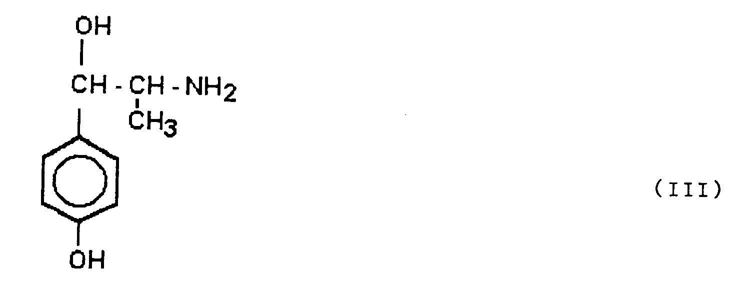

2. Le procédé selon la revendication 1, dans lequel l'amination réductrice est effectuée en utilisant des hydrures métalliques.

3. Le procédé selon la revendication 2, dans lequel on utilise comme hydrures métalliques le cyanoborohydrure de sodium ou un mélange de cyanoborohydrure de sodium et de cyanoborohydrure de tétrabutylammonium dans un rapport en poids de 4:1 à 1:1.

4. Procédé de préparation des composés de formule (I) 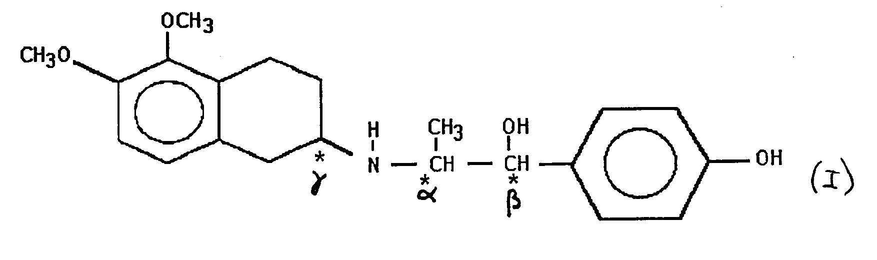 comprenant la réduction d'un composé de formule (VI) 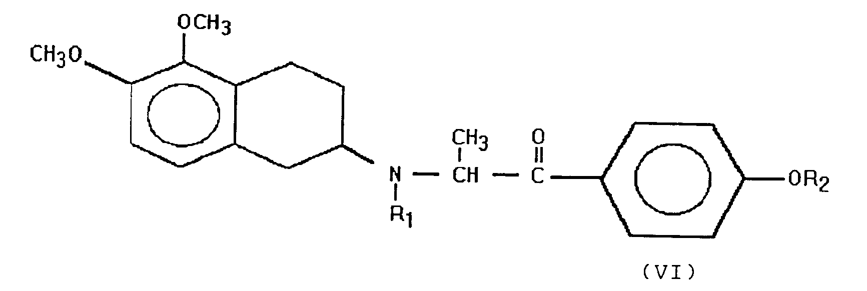 dans laquelle R₁ et R₂ sont l'hydrogène ou un groupe benzyle ou un groupe présentant un empêchement stérique similaire, groupe qui peut ensuite être éliminé par des méthodes connues en elles-mêmes.
